# EUROPEAN PATENT SPECIFICATION

(11) **EP 0 509 009 B1**
(45) Date of publication and mention of the grant of the patent: **19.04.1995**
(21) Application number: 91901713.7
(22) Date of filing: 21.12.1990
(51) Int. Cl.: A61K 31/23, A61K 31/22, A61K 9/107, C07C 69/30

(54) **USE OF A MATERIAL, THE MAIN CONSTITUENT OF WHICH IS A TRIGLYCERIDE, AS AN AGENT WITH BIOLOGICAL EFFECT ON THE INTESTINAL MUCOSA AND PREPARATION CONTAINING SUCH AGENT**
VERWENDUNG EINES STOFFES, DESSEN HAUPTBESTANDTEIL EIN TRIGLYZERID IST, ALS WIRKSTOFF MIT BIOLOGISCHER WIRKUNG AUF DIE DARMMUCOSA SOWIE ZUBEREITUNG DIE DIESEN WIRKSTOFF ENTHÄLT
UTILISATION D'UNE SUBSTANCE, DONT LE CONSTITUANT PRINCIPAL EST UNE TRIGLYCERIDE, COMME AGENT PRESENTANT UN EFFET BIOLOGIQUE SUR LES MUQUEUSES INTESTINALES ET PREPARATION CONTENANT CET AGENT

(30) Priority: 22.12.1989 DK 6576/89
(43) Date of publication of application: 21.10.1992
(73) Proprietor: NOVO NORDISK A/S, 2880 Bagsvaerd (DK)
(72) Inventor: FROEKJAER, Sven, DK-2850 Naerum (DK); HANSEN, Tomas, Tage, DK-3450 Alleroed (DK)
(86) International application number: DK9000343
(87) International publication number: WO9109597

(56) References cited:
- EP-A- 0 265 699
- EP-A- 0 300 844
- DE-A- 407 180
- DE-A- 1 418 149
- US-A- 4 272 548
- US-A- 4 701 469

## Description

The invention comprises the use of an agent, of which the main constituent is a triglyceride for the manufacture of a medicament for the prevention of the development and the reconstitution of atrophic mucosa.

The prior art agents with such biological effect on the intestinal mucosa are either enteral or parenteral. The prior art enteral agents, which reconstitute atrophic mucosa and prevent development of atrophic mucosa, are either fibres (vide e.g. John H. Cummings, The Role of Dietary Fiber in Enteral Nutrition, Abbott International LTD, Abbott Park, Illinois 60064) or free fatty acids (vide the Review Bacterial short-chain fatty acids and mucosal diseases of the colon, Br. J. Surg. 1988, vol. 75, April, 346-348). The fibres exhibit the disadvantage comprising generation of voluminosity in the faeces, and for that reason low residue diets have been developed. The free fatty acids exhibit the drawback of a bad smell and a bad tacte. Furthermore the free fatty acids are subject to the so-called first pass effect i.e. they are rapidly eliminated by the liver after being absorbed from the intestines into the blood stream, and thus, they will not be circulated in the blood stream and will thus only exert a minor effect on the intestinal mucosa. The prior art parenteral agents, which reconstitute atrophic mucosa and prevent development of atrophic mucosa, are free fatty acids (vide Koruda et al., Gastroenterology 1988; 95:715-20, Effect of Parenteral Nutrition Supplemented With Short-Chain Fatty Acids on Adaptation to massive Small Bowel Resection), but with parenteral administration the free fatty acids develop acidosis.

Thus the purpose of the invention is the provision of a use of an agent for the manufacture of a medicament with the indicated biological effect on the intestinal mucosa, which medicament is not accompanied by the above indicated drawbacks.

Now, surprisingly according to the invention it has been found that the use of an agent, of which the main constituent is a triglyceride belonging to a specific group of triglycerides is able to fulfil this purpose.

Thus, the invention comprises the use of an agent, of which the main constitutent is a triglyceride which contains both one or more linear, saturated or unsaturated acyl groups containing 2 - 5 carbon atoms, and one or more linear, saturated or unsaturated acyl groups containing 16 - 24 carbon atoms, for the manufacture of a medicament for the prevention of the development and the reconstitution of atrophic mucosa.

The above indicated triglycerides are described per se in DE 1418149, but it does not appear from DE 1418149 that they can be used as agents with the indicated biological effect on the intestinal mucosa.

Surprisingly it has been found that the medicament prepared by the use according to the invention does not exhibit any of the drawbacks which accompany prior art agents with such biological effect on the intestinal mucosa. Furthermore besides their capacity as agents with the indicated biological effect on the intestinal mucosa, they are able to function as emulsifiers, and thus, they can be introduced into the organism as emulsifiers for nutritional fats and thus at the same time perform two distinct and separate functions.

In a preferred embodiment of the use according to the invention the triglyceride has the general formula 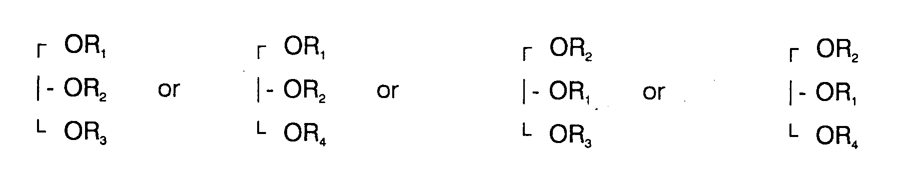
wherein R₁ and R₃ are identical or different, each representing a linear, saturated or unsaturated acyl group containing 2 - 5 carbon atoms, and wherein R₂ and R₄ are identical or different, each representing a linear, saturated or unsaturated alkyl group containing 16 - 24 carbon atoms. The medicament prepared by this embodiment of the use according to the invention exhibits both an excellent biological effect of the indicated kind on the intestinal mucosa and an excellent effect as an emulsifier.

In a preferred embodiment of the use according to the invention the medicament is an enteral aqueous fat emulsion, in which the agent constitutes 1 - 100% of the total fat.

In a preferred embodiment of the use according to the invention, wherein the medicament is an enteral aqueous fat emulsion, in which the agent in the medicament constitutes less than 100% of the total fat, the non agent fatty material is soy oil, sunflower oil, safflower oil, canola oil, fish oil, or MCT oils. Other examples are the structured lipids which are described in WO 90/4008, 90/4009, 90/04010, 90/03786, 90/03787, 90/04011, 90/04012, and 90/04013, all from Novo Nordisk A/S, Denmark. Another embodiment of the enteral medicament in the use according to the inventon can be formulated as indicated in Example 1 of European patent specification EP 0 246 747, except for the fact that necessary vitamins and minerals are added. These embodiments offer the possibility for production of a fat which is nutritionally balanced.

In a preferred embodiment of the use according to the invention the medicament is for parenteral use, and the agent constitutes 1-100% of the fat source.

In a preferred embodiment of the use according to the invention wherein the medicament is parenteral the average diameter of the fat globules is between 5 and 1500 nm, less than 5% of the fat globules exhibit a diameter above 5000 nm, and the preparation contains approx. 2-3% glycerol. Glycerol is added in such amount that the preparation is isotonic. Other isotonicity generating agents can be used. Also, other auxiliary agents, such as pH regulating agents, can be added.

EP-A-300,844 describes mixed triglycerides falling within the scope of the triglycerides indicated in relation to the use according to the invention and mentions their use as dietetic agent or in cardiovascular therapy. This use, however, is not related whatsoever to a medicament for the prevention of the development and the reconstitution of atrophic mucosa.

US-A-4,272,548 describes mixed triglycerides falling within the scope of the triglycerides indicated in relation to the use according to the invention and which are used for lowering increased cholesterol and/or increased neutral fat levels in blood. This use, however, is not related whatsoever to a medicament for the prevention of the development and the reconstitution of atrophic mucosa.

US-A-4,701,469 also describes mixed triglycerides falling within the scope of the triglycerides indicated in relation to the use according to the invention and mentions pharmacological properties such as anti-aggregation or immuno-modulating properties. These pharmacological properties, however, are not related whatsoever to a medicament for the prevention of the development and the reconstitution of atrophic mucosa.

Example 1 illustrates the synthesis of a triglyceride within the scope of Claims 1 and 2, Examples 2 and 3 illustrate the production of a use according to Claim 3, whereby Example 2 is related to Claim 6 and Example 3 is related to Claim 4, and Example 4 illustrates the effect of a perorally administered medicament made by the use according to the invention on gut mucosal growth in rats.

### EXAMPLE 1

### Synthesis of a lipid containing both butyric acid and long chain acids

30 g of a high oleic sunflower oil and 6 g of butyric acid dissolved in 150 ml heptane was shaken together with 3 g of Lipozyme (a commercially available lipase from Novo Nordisk A/S) at 40°C for 24 hours. Thereafter the immobilized lipase was filtered off and the mixture was introduced onto a column containing 600 g of activated alkaline Al₂O₃, and the column was eluted with 800 ml of diethyl ether. The diethyl ether was removed by evaporation under vacuum. The remaining lipid was 100% triglycerides, i.e. no mono- or diglyceride was present. The fatty acid composition was analyzed by conversion of the fatty acids to butyl esters, catalyzed by sodium butylate dissolved in butanol, and injection in a capillary gas chromatograph. The fatty acid composition of the lipid was as follows:

| acids | weight | moles |
|---|---|---|
| C-4 | 22.0% | 47.4% |
| C-16:0 | 2.0% | 1.5% |
| C-18:0 | 3.6% | 2.4% |
| C-18:1¹ | 70.6% | 47.5% |
| C-18:1² | 1.9% | 1.3% |

For later reference this lipid is identified by the term lipid 89.034.

### EXAMPLE 2

A parenteral diet product is prepared according to the following formula:

### Composition for 100 ml

| | |
|---|---|
| Lipid 89.034 | 1.2 g |
| Glycerol | 2.25 g |
| Soy oil | 10.0 g |
| Water | to 100 ml |

For the preparation of one liter of product the procedure is as follows:

### Constituents

| | | |
|---|---|---|
| I | Lipid 89.034 | 12.0 g |
| | Soy oil | 100.0 g |
| II | Glycerol | 22.5 g |
| | Water for injection | to 1000 ml |

### Production procedure

I is mixed with II in a high shear mixer (Ultra Turrax). The pre-emulsion is homogenized in a high pressure homogenizer and then introduced into a suitable container. The product is finally pasteurized at 85°C for 10 minutes.

### Analytical data

| | |
|---|---|
| Particle size: | 1.2 »m |
| Osmolality: | 260 mOsm/kg water |
| pH: | 7.1 |

### EXAMPLE 3

An enteral diet product is prepared according to the following formula:

### Composition for 100 ml

| | | |
|---|---|---|
| Protein: | Soy protein hydrolysate (Nx6.25) | 3.75 g |
| Carbohydrate: | Maltodextrins | 13.75 g |
| Fat: | Medium chain triglycerid | 1.73 g |
| | Corn oil | 1.00 g |
| | Soy bean oil | 0.35 g |
| | Lipid 89.034 | 0.39 g |
| Water: | | to 100 ml |

For the preparation of one liter of product the procedure is as follows:

### Constituents

| | | |
|---|---|---|
| I | Tri-calcium dicitrate, 4H₂O | 1.044 g |
| | Tri-sodium citrate, 2H₂O | 1.84 g |
| | Tri-potassium citrate, H₂O | 2.42 g |
| | Citric acid, H₂O | 2.75 g |
| | Methionine | 0.48 g |
| | Tryptophan | 0.16 g |
| | Ascorbic acid | 0.30 g |
| | Aspartam | 0.315 g |
| | Vitamin premix | 0.10 g |
| II | Soy protein hydrolyzate | 37.0 g |
| | Water | 200 ml |
| III | Maltodextrin | 137.5 g |
| | Pectin | 2.5 g |
| | Water | 400 ml |
| IV | Viscoleo | 17.3 g |
| | Corn oil | 10.0 g |
| | Lipid 89.034 | 3.9 g |
| | Soy oil | 3.5 g |
| V | Water | to 1000 ml |

### Production procedure

I is dissolved in II. pH is measured to pH = 4.3. Maltodextrin and pectin are dissolved in water by heating to 50°C, III is mixed with I + II and IV is added. The product is homogenized on a high pressure homogenizer. The product is introduced into a suitable container and pasteurized at 85°C for 10 minutes.

### Analytical data

| | |
|---|---|
| Particle size: | 0.66 »m |
| Osmolality: | 266 mOsm/kg water |
| pH: | 4.3 |

### EXAMPLE 4

This example illustrates the effect of perorally administered SLS on gut mucosal growth in rats, SLS being an abbreviation for a structured lipid with butyrate in 1 and 3 positions and long chain fatty acids from soy bean oil in the 2 position.

30 Sprague Dawley rats (average 300 g) were randomly assigned to receive one of the following diets:
1) Standard rat Chow
2) Elemental diet and maltodextrin
3) Elemental diet and structured lipid emulsion (SLS)
The elemental diets were Vivasorb from Pfrimmer. Diets 1) - 3) were provided in isocaloric amounts (230 kcal/kg body wt/day) for 8 days. The lipid content in diet 3) was 5% by weight. All diets contained choline chloride (194 »g/kcal).

At day 8 the animals were anesthetized with CO₂/O₂ mixture and exsanguinated, and the intestine was rapidly excised. 10 cm segments from the proximal jejunum and colon were obtained, opened longitudinally, rinsed in saline solution and scraped with a scalpel for complete removal of mucosa. The mucosal scrapings were weighed, freeze-dried and frozen at -20°C until analysis for protein¹ and DNA². In addition 2 cm segments of the proximal jejunum and colon were excised and fixated in formalin for morphometric analysis.

The dietary effect on the jejunal mucosal dry weight, protein and DNA content appears from the following table.

| Diets | Mucosal DNA (»g/cm) | Mucosal weight (mg/cm) | Mucosal protein (mg/cm) |
|---|---|---|---|
| 1) Chow | 384.4 ± 38.9 | 11.6 ± 0.90 | 5.57 ± 0.45 |
| 2) Maltodextrin + Vivasorb | 392.5 ± 41.1 | 10.5 ± 0.39 | 4.31 ± 0.32 |
| 3) SLS + Vivasorb | 479.8 ± 41.6 | 11.5 ± 0.33 | 4.74 ± 0.37 |
| Values are average of 10 independent determinations ± SEM | | | |

Chow 1) is the normal, fibre containing diet. In case of some diseases, however, the normal fibre containing diet can not be used, and a fibre free, fluid diet has to be used. Thus, the diet 2) is the prior art diet most related to the diet according to the invention, 3).

It appears from the above table that the diet according to the invention in all regards corresponding to the heads of the last three columns are superior to the prior art diet most related to the diet according to the invention. Also, the diet according to the invention comes close to the normal fibre containing diet in regard to mucosal protein, it comes very close to the normal fibre containing diet in regard to mucosal weight, and it supersedes the normal fibre containing diet in regard to mucosal DNA, thus indicating that the mitotic activity generated by the diet according to the invention is very high.

### References:

1. Lowry OM, Rosebrough NJ, Farr AL and Randall R: Protein measurement with the folin phenol reagent. J.Biol.Chem. 193: 265-275, 1951
2. Cesarone CF, Bolognesi C and Santi L: Improved microgluorometric DNA determination in biological material using 33258 Hoechst. Anal.Biochem. 100: 188-197, 1979

## Claims

1. Use of an agent, of which the main constitutent is a triglyceride which contains both one or more linear, saturated or unsaturated acyl groups containing 2 - 5 carbon atoms, and one or more linear, saturated or unsaturated acyl groups containing 16 - 24 carbon atoms, for the manufacture of a medicament for the prevention of the development and the reconstitution of atrophic mucosa.

2. Use according to Claim 1, wherein the triglyceride has the general formula 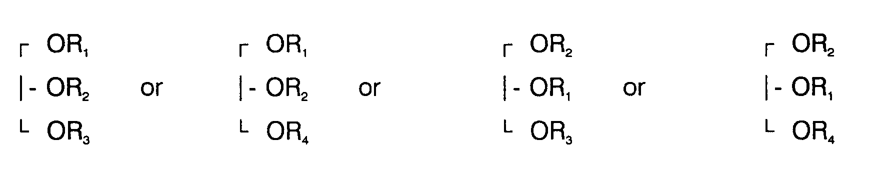 wherein R₁ and R₃ are identical or different, each representing a linear, saturated or unsaturated acyl group containing 2 - 5 carbon atoms, and wherein R₂ and R₄ are identical or different, each representing a linear, saturated or unsaturated alkyl group containing 16 - 24 carbon atoms.

3. Use according to Claims 1 - 2, wherein the medicament is an enteral aqueous fat emulsion, in which the agent constitutes 1-100% of the total fat.

4. Use according to Claim 3, in which the agent in the medicament constitutes less than 100% of the total fat, wherein the non agent fatty material is soy oil, sunflower oil, safflower oil, canola oil, fish oil, or MCT oils.

5. Use according to Claims 1 - 2, wherein the medicament is parenteral and the agent constitutes 1-100% of the fat source.

6. Use according to Claim 5, wherein the average diameter of the fat globules is between 5 and 1500 nm, wherein less than 5% of the fat globules exhibit a diameter above 5000 nm, and wherein the preparation contains 2-3% glycerol.

## Patentansprüche

1. Verwendung eines Mittels, dessen Hauptbestandteil ein Triglycerid ist, das sowohl eine oder mehrere lineare, gesättigte oder ungesättigte Acylgruppen mit 2 bis 5 Kohlenstoffatomen als auch eine oder mehrere lineare, gesättigte oder ungesättigte Acylgruppen mit 16 bis 24 Kohlenstoffatomen enthält, zur Herstellung eines Arzneimittels für die Verhinderung der Entwicklung und zur Wiederherstellung einer atrophen Schleimhaut.

2. Verwendung nach Anspruch 1, wobei das Triglycerid die allgemeine Formel 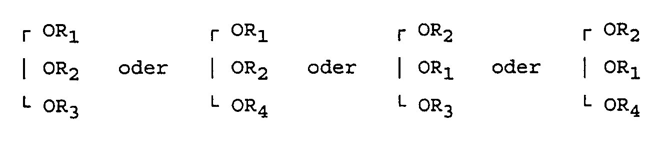 aufweist, worin R₁ und R₃ gleich oder verschieden sind und jeweils eine lineare, gesättigte oder ungesättigte Acylgruppe mit 2 bis 5 Kohlenstoffatomen darstellen und worin R₂ und R₄ gleich oder verschieden sind und jeweils eine lineare, gesättigte oder ungesättigte Acylgruppe mit 16 bis 24 Kohlenstoffatomen darstellen.

3. Verwendung nach Anspruch 1 oder 2, wobei das Arzneimittel eine enterale wäßrige Fettemulsion ist, in der das Mittel 1 bis 100 % des Gesamtfetts ausmacht.

4. Verwendung nach Anspruch 3, wobei das Mittel in dem Arzneimittel weniger als 100 % des Gesamtfetts ausmacht, wobei es sich bei dem von dem Mittel verschiedenen Fettmaterial um Sojabohnenöl, Sonnenblumenöl, Safloröl, Canolaöl, Fischöl oder MCT-Öle handelt.

5. Verwendung nach einem der Ansprüche 1 oder 2, wobei das Arzneimittel parenteral ist und das Mittel 1 bis 100 % der Fettquelle ausmacht.

6. Verwendung nach Anspruch 5, wobei der mittlere Durchmesser der Fettkügelchen zwischen 5 und 1500 nm beträgt, wobei weniger als 5 % der Fettkügelchen einen Durchmesser von mehr als 5000 nm aufweisen und wobei die Zubereitung 2 bis 3 % Glycerin enthält.

## Revendications

1. L'utilisation d'un agent dont le constituant principal est un triglycéride qui contient à la fois un ou plusieurs groupes acyle linéaires saturés ou insaturés renfermant de 2 à 5 atomes de carbone et un ou plusieurs groupes acyle linéaires, saturés ou insaturés, contenant de 16 à 24 atomes de carbone pour la fabrication d'un médicament pour empêcher le développement et pour la reconstitution de la muqueuse atrophique.

2. L'utilisation selon la revendication 1, dans laquelle le triglycéride a la formule générale : 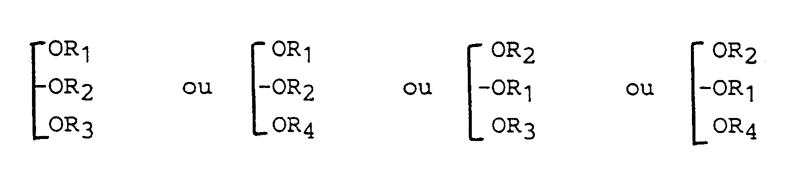 où R₁ et R₃ sont identiques ou différents et représentent chacun un groupe acyle linéaire saturé ou insaturé renfermant de 2 à 5 atomes de carbone et où R₂ et R₄ sont identiques ou différents, représentant chacun un groupe acyle linéaire saturé ou insaturé contenant de 16 à 24 atomes de carbone.

3. L'utilisation selon les revendications 1-2, dans laquelle le médicament est une émulsion de graisse aqueuse entérale où l'agent représente de 1 à 100% de la graisse totale.

4. L'utilisation selon la revendication 3, dans laquelle l'agent dans le médicament représente moins de 100% de la graisse totale où la matière grasse qui n'est pas l'agent est l'huile de soja, l'huile de tournesol, l'huile de carthame, l'huile de canola, l'huile de poisson ou les huiles MCT.

5. L'utilisation selon les revendications 1-2, dans laquelle le médicament est parentéral et l'agent représente de 1 à 100% de la source de graisse.

6. L'utilisation selon la revendication 5, dans laquelle le diamètre moyen des globules de graisse se situe entre 5 et 1500 nm, où moins de 5% des globules de graisse présentent un diamètre supérieur à 5000 nm, et où la préparation renferme 2 à 3% de glycérol.
